# EUROPEAN PATENT APPLICATION

(11) **EP 1 947 097 A1**
(43) Date of publication of application: **23.07.2008**
(21) Application number: 06822953.3
(22) Date of filing: 27.10.2006
(51) Int. Cl.: C07D 333/56, C07C 333/04, C07D 333/62, C07D 409/06, C07D 409/14, C07D 471/04

(54) **BENZOÝb¨THIOPHEN DERIVATIVE AND PROCESS FOR PRODUCTION THEREOF**

(30) Priority: 27.10.2005 JP 2005312697
(71) Applicant: Teijin Pharma Limited, Chiyoda-ku, Tokyo 100-0013 (JP)
(72) Inventor: YAJIMA, Naoki, Iwakuni-shi Yamaguchi 740-0014 (JP); SATO, Yoshinori, Iwakuni-shi Yamaguchi 740-0014 (JP); YOSHINO, Hiroshi, Ichikawa-shi Chiba 272-0823 (JP); KOIZUMI, Tatsuya, Narashino-shi Chiba 275-0016 (JP); HIROKI, Yasuhiro, Chiba-shi Chiba 261-0003 (JP)
(74) Representative: Hallybone, Huw George
(86) International application number: PCT/JP2006/322035
(87) International publication number: WO 2007/049813

(57) **Abstract**

The invention provides benzo[b]thiophene derivatives useful as production intermediates for chymase inhibitors, and a process for their production.

The invention relates to benzo[b]thiophene derivatives represented by the following formula, and a process for their production.

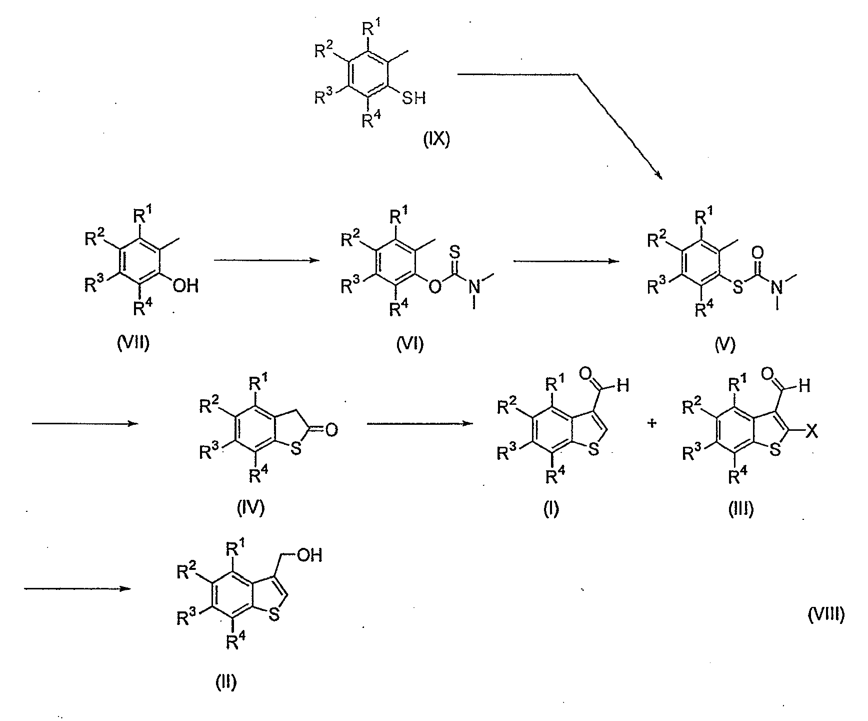

## Description

### Technical Field

The present invention relates to a process for producing 3-hydroxymethylbenzo[b]thiophene derivatives that are important as production intermediates for compounds useful as drugs. More specifically, it relates to a process for producing production intermediates that are useful for synthesis of chymase inhibitors which can be applied as prophylactic and/or therapeutic agents for a variety of conditions including respiratory diseases such as bronchial asthma, sclerotic vascular lesions, vascular constriction and peripheral circulatory organ disorders.

### Background Art

Formula (II): 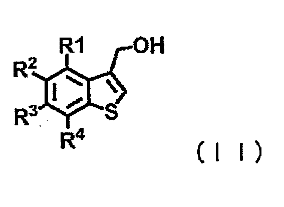 wherein R¹, R², R³ and R⁴ simultaneously or each independently represent a hydrogen atom, C1-6 alkyl, C1-6 halogenated alkyl, a halogen atom, cyano, C1-6 alkoxy, C1-6 alkylthio, C1-6 acyloxy, C1-6 acylamino or C1-6 halogenated alkoxy.

The 3-hydroxymethylbenzo[b]thiophene derivatives represented by formula (II) above are of great importance as intermediates in the production of pharmacologically active compounds. For example, compounds wherein the hydroxyl group in compounds represented by formula (II) above is substituted with bromine can serve as synthetic intermediates for the benzimidazole derivatives disclosed in International Patent Publication No. WO 01/53291, and are very important as intermediates for production of pharmacologically active compounds. These benzimidazole derivatives have chymase inhibiting activity in the body, and are promising as compounds with applications as prophylactic or therapeutic agents for inflammatory diseases, allergic diseases, respiratory diseases, circulatory diseases or bone/cartilage metabolic disorders.

So far, it has been extremely difficult to produce benzothiophene derivatives having a hydroxymethyl group at the 3-position and regioselective placement of other substituents, as in the compounds represented by formula (II) above, and their industrial production has not been feasible. For example, existing processes synthesize 3-formyl-benzo[b]thiophenes by Vilsmeier reaction of benzo[b]thiophene (J. Org. Chem., 72, 1422, (1957)) followed by their reduction, or synthesize 3-trichloroacetyl-benzo[b]thiophene derivatives as starting materials by Friedel-Crafts reaction of benzo[b]thiophene (J. Chem. Soc., Perkin Trans. 2, 1250, (1973)) followed by their hydrolysis and subsequent reduction. However, in all such processes, substitution reaction proceeds either at positions 2 and 3 or at any position(s) of positions 2 to 7 on the benzo[b]thiophene ring, depending on the type and positions of the substituents that are initially present. The selectivity is not very high and tends to depend on the substrate used and the reaction conditions. Also, it is extremely difficult to isolate only the target compound from the resulting mixture.

International Patent Publication No. WO 02/066457 and International Patent Publication No. WO 02/090345 disclose examples of production processes for obtaining benzothiophene derivatives having a hydroxymethyl group at the 3-position similar to the compounds represented by formula (II) above, with regioselective placement of other substituents.

However, production processes with higher yields than the process described in International Patent Publication No. WO 02/066457 and with shorter steps than the process described in International Patent Publication No. WO 02/090345 are desired. High yields with short production steps are major advantages for large-scale synthesis.

### DISCLOSURE OF THE INVENTION

It is an object of the present invention to provide a process suitable for regioselective, high-yield and more rapid production of 3-hydroxymethyl-4-methylbenzo[b]thiophene which is useful as a production intermediate for the chymase inhibiting compounds described in International Patent Publication No. WO 01/53291.

As a result of much diligent research directed toward achieving the aforestated object, the present inventors have discovered a regioselective process for production of 3-hydroxymethylbenzo[b]thiophene derivatives with shorter steps and higher yield than the

### prior art.

Specifically, the invention provides the following.
(1) A process wherein a compound represented by the following formula (I): 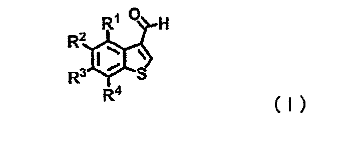 wherein R¹, R², R³ and R⁴ simultaneously or each independently represent a hydrogen atom, C1-6 alkyl, C1-6 halogenated alkyl, a halogen atom, cyano, C1-6 alkoxy, C1-6 alkylthio, C1-6 acyloxy, C1-6 acylamino or C1-6 halogenated alkoxy;
   is reduced to produce a 3-hydroxymethylbenzo[b]thiophene derivative represented by the following formula (II) : 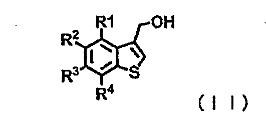 wherein R¹, R², R³ and R⁴ are as defined in formula (I).
(2) The production process according to (1), wherein a metal hydride complex is used as the reducing agent.
(3) The production process according to (2), wherein the metal hydride complex is sodium borohydride complex or aluminum hydride complex.
(4) A process wherein a compound represented by the following formula (III): 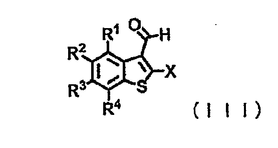 wherein R¹, R², R³ and R⁴ are as defined in formula (I), and X represents a halogen atom;
   is reduced to produce a 3-hydroxymethylbenzo[b]thiophene derivative represented by formula (II).
(5) The production process according to (4), wherein a metal hydride complex is used as the reducing agent.
(6) The production process according to (5), wherein the metal hydride complex is an aluminum hydride complex.
(7) The production process according to any one of (1) to (3), wherein the compound represented by formula (III) is dehalogenated to produce a 3-formylbenzo[b]thiophene derivative represented by formula (I).
(8) The production process according to (7), wherein catalytic reduction is used as the dehalogenation conditions.
(9) The production process according to (8), wherein hydrogen and palladium-carbon are used as the dehalogenation reagents.
(10) A process wherein a mixture of a compound represented by formula (I) and a compound represented by formula (III) is reduced to produce a 3-hydroxymethylbenzo[b]thiophene derivative represented by formula (II).
(11) The production process according to (10), wherein a metal hydride complex is used as the reducing agent.
(12) The production process according to (11), wherein the metal hydride complex is an aluminum hydride complex.
(13) The production process according to (12), wherein the aluminum hydride complex is sodium bis(2-methoxyethoxy)aluminum hydride.
(14) The production process according to any one of (1) to (3) and (10) to (13), wherein a compound represented by the following formula (IV): 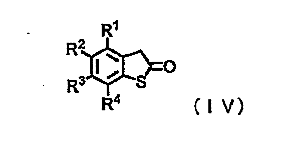 wherein R¹, R², R³ and R⁴ are as defined in formula (I);
   is formylated to produce a 3-formylbenzo[b]thiophene derivative represented by formula (I).
(15) The production process according to (14), wherein Vilsmeier reaction conditions are used as the formylation conditions.
(16) The production process according to (14), wherein N,N-dimethylformamide and phosphorus oxychloride are used as the formylation reagents.
(17) The production process according to any one of (4) to (13), wherein a compound represented by formula (IV) is formylated to produce a 3-formylbenzo[b]thiophene derivative represented by formula (III).
(18) The production process according to (17), wherein Vilsmeier reaction conditions are used as the formylation conditions.
(19) The production process according to (17), wherein N,N-dimethylformamide and phosphorus oxychloride are used as the formylation reagents.
(20) The production process according to any one of (14) to (19), wherein a compound represented by the following formula (V): 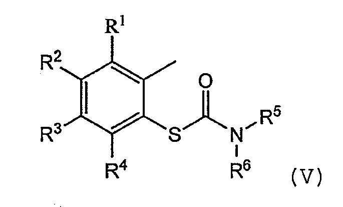 wherein R¹, R², R³ and R⁴ are as defined in formula (I), and R⁵ and R⁶ simultaneously or each independently represent a hydrogen atom, C1-6 alkyl, C1-6 halogenated alkyl, a halogen atom, cyano, C1-6 alkoxy, C1-6 alkylthio, C1-6 acyloxy, C1-6 acylamino or C1-6 halogenated alkoxy;
   is cyclized to produce a dihydrobenzothiophene derivative represented by formula (IV).
(21) The production process according to (20), wherein a base is used as the cyclizing reagent.
(22) The production process according to (21), wherein lithium diisopropylamide is used as the cyclizing reagent.
(23) The production process according to any one of (20) to (22), wherein a compound represented by formula (IX): 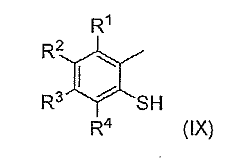 wherein R¹, R², R³ and R⁴ are as defined as in formula (I);
   is reacted with a carbamoyl halide to produce a carbamoyl derivative represented by formula (V).
(24) The production process according to (23), wherein a base is used as the reagent.
(25). The production process according to (24), wherein potassium carbonate or sodium hydride is used as the reagent.
(26) The production process according to any one of (20) to (22), wherein a compound represented by formula (VI): 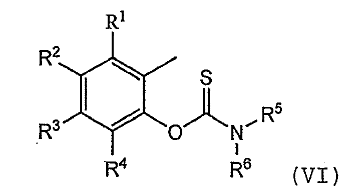 wherein R¹, R², R³, R⁴, R⁵ and R⁶ are as defined in formula (V);
   is subjected to rearrangement reaction to produce a thiocarbamoyl derivative represented by formula (V).
(27) The production process according to (26), wherein the rearrangement reaction conditions include heating at a temperature of between 200°C and 300°C.
(28) The production process according to (26) or (27), wherein a compound represented by formula (VII): 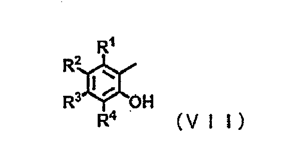 wherein R¹, R², R³ and R⁴ are as defined in formula (I);
   is reacted with a thiocarbamoyl halide to produce a carbamoyl derivative represented by formula (VI).
(29) A production process for benzimidazole derivatives represented by formula (XX), which includes the following production steps:
   (i) a production process for a compound represented by formula (II), according to any one of (1) to (28); and
   (ii) a process in which a benzimidazole derivative represented by formula (XX) is produced from the compound represented by formula (II); 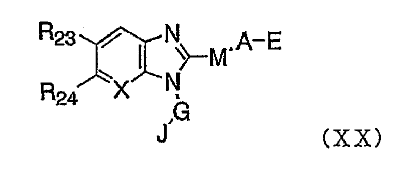
   in formula (XX)
   R₂₃ and R₂₄ simultaneously or each independently represent a hydrogen atom, a halogen atom, trihalomethyl, cyano, hydroxyl, C1-4 alkyl or C1-4 alkoxy, or R₂₃ and R₂₄ may together form -O-CH₂O-, -O-CH₂CH₂O- or -CH₂CH₂CH₂- (in which case the carbon atoms may be optionally substituted with one or more C1-4 alkyl groups);
   A represents a substituted or unsubstituted C1-7 straight-chain, cyclic or branched alkylene group or alkenylene group, which may include one or more group(s) selected from the group consisting of -O-, -S-, -SO₂- and -NR₂₅- (where R₂₅ represents a hydrogen atom or a straight-chain or branched C1-6 alkyl group), and the substituent(s) on the groups may be a halogen atom or hydroxyl, nitro, cyano, straight-chain or branched C1-6 alkyl or straight-chain or branched C1-6 alkoxy groups (including cases where two adjacent groups form an acetal bond), straight-chain or branched C1-6 alkylthio, straight-chain or branched C1-6 alkylsulfonyl, straight-chain or branched C1-6 acyl, straight-chain or branched C1-6 acylamino, trihalomethyl, trihalomethoxy, phenyl, oxo or phenoxy optionally substituted with one or more halogen atom(s), and one or more of the substituents at any desired position of the alkylene or alkenylene group may be independently substituted, with the exclusion of cases where M in formula (XX) is a single bond and hydroxyl and phenyl groups simultaneously substitute carbons of A bonded to M;
   E represents -COOR₂₅, -SO₃R₂₅, -CONHR₂₅, -SO₂NHR₂₅, tetrazol-5-yl, 5-oxo-1,2,4-oxadiazol-3-yl or 5-oxo-1,2,4-thiadiazol-3-yl (where R₂₅ is as defined above);
   M represents a single bond or -S(O)m-, where m is an integer of 0-2;
   G and J together represent formula (II) above, with G representing the methylene group at position 3 of the benzothiophene of formula (II), and the hydroxyl group of formula (II) being substituted by the nitrogen atom on the benzimidazole ring; and
   X represents -CH= or a nitrogen atom.
(30) A compound represented by the following formula (I): 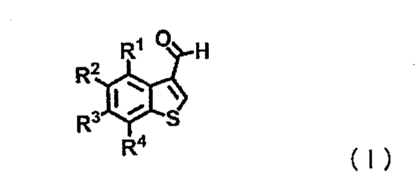 wherein R¹, R², R³ and R⁴ are as defined above.
(31) The compound according to (30), wherein three among R¹, R², R³ and R⁴ are hydrogen atoms.
(32) The compound according to (31), wherein R², R³ and R⁴ are hydrogen atoms.
(33) The compound according to (32), wherein R¹ is a C1-6 alkyl group.
(34) The compound according to (33) wherein R¹ is a methyl group.
(35) A compound represented by the following formula (III): 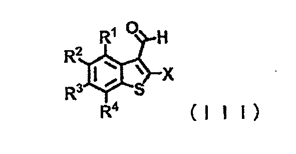 wherein R¹, R², R³ and R⁴ are as defined in formula (I).
(36) The compound according to (35), wherein three from among R¹, R², R³ and R⁴ are hydrogen atoms.
(37) The compound according to (36), wherein R², R³ and R⁴ are hydrogen atoms.
(38) The compound according to (37), wherein R¹ is a C1-6 alkyl group.
(39) The compound according to (38), wherein R¹ is a methyl group and X is a chlorine atom.
(40) A compound represented by the following formula (V): 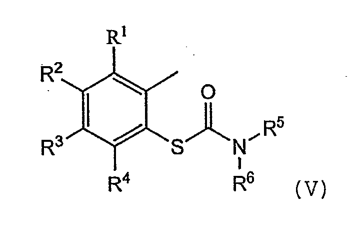 wherein R¹, R², R³, R⁴, R⁵ and R⁶ are as defined above.
(41) The compound according to (40), wherein R⁵ and R⁶ simultaneously or each independently are methyl or ethyl groups.
(42) The compound according to (41), wherein three from among R¹, R², R³ and R⁴ are hydrogen atoms.
(43) The compound according to (42), wherein R², R³ and R⁴ are hydrogen atoms.
(44) The compound according to (43), wherein R¹ is a C1-6 alkyl group.
(45) The compound according to (44), wherein R¹ is a methyl group.
(46) A compound represented by the following formula (VI) : 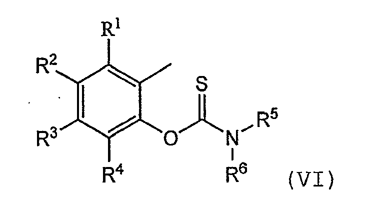 wherein R¹, R², R³, R⁴, R⁵ and R⁶ are as defined in formula (V).
(47) The compound according to (46), wherein R⁵ and R⁶ simultaneously or each independently are methyl or ethyl groups.
(48) The compound according to (47), wherein three from among R¹, R², R³ and R⁴ are hydrogen atoms.
(49) The compound according to (48), wherein R², R³ and R⁴ are hydrogen atoms.
(50) The compound according to (49), wherein R¹ is a C1-6 alkyl group.
(51) The compound according to (50), wherein R¹ is a methyl group.

The products of the production process of the invention may form salts, and produced salts or salts derived after the reaction are also encompassed within the scope of the invention.

The invention allows 3-hydroxymethylbenzo[b]thiophene derivatives that are useful as production intermediates for chymase inhibitor compounds to be produced with short steps and a high yield in a regioselective manner, and therefore has high industrial value.

### Best Mode for Carrying Out the Invention

The production process of the invention may be summarized by chemical formula (VIII) below. However, the invention may also be constituted by any one of the individual steps alone.
Formula (VIII): 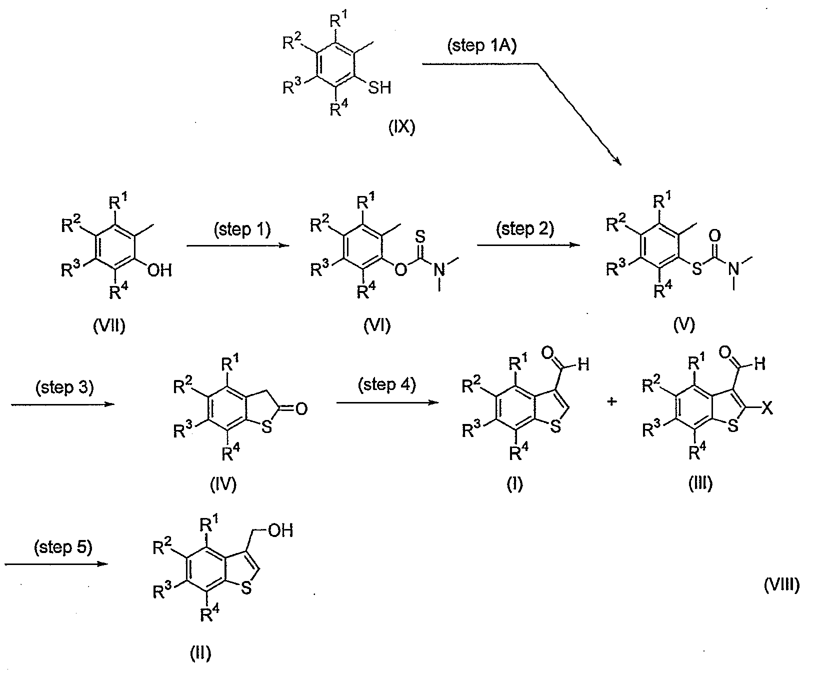 wherein R¹, R², R³, R⁴, R⁵ and R⁶ simultaneously or each independently represent a hydrogen atom, C1-6 alkyl, C1-6 halogenated alkyl, a halogen atom, cyano, C1-6 alkoxy, C1-6 alkylthio, C1-6 acyloxy, C1-6 acylamino or C1-6 halogenated alkoxy, and X represents a halogen atom.

In the production process of the invention, any three from among R¹, R², R³ and R⁴ are preferably hydrogen atoms, and preferably R², R³ and R⁴ are hydrogen atoms. In this case, R¹ is preferably a C1-4 alkyl group, and most preferably R¹ is a methyl group. X is preferably a chloro group. Preferably, R⁵ and R⁶ are simultaneously or each independently a C1-4 alkyl group, and are most preferably methyl or ethyl groups.

As used herein, the term "C1-6 alkyl" group refers to a C1-6 straight-chain or branched alkyl group. As examples there may be mentioned methyl, ethyl, n-propyl, n-butyl, n-pentyl, n-hexyl, n-heptyl, n-octyl, isopropyl, isobutyl, sec-butyl, tert-butyl, isopentyl, neopentyl, tert-pentyl, isohexyl, 2-methylpentyl and 1-ethylbutyl.

As used herein, the term "halogen atom" refers to fluorine, chlorine, bromine, iodine and the like, among which fluorine, chlorine and bromine may be mentioned as preferred examples.

As used herein, the term "C1-6 halogenated alkyl" refers to a group comprising a halogen atom and the aforementioned "C1-6 alkyl" group. As examples there may be mentioned methyl fluoride, methyl chloride, methyl bromide, 1-ethyl fluoride, 2-ethyl fluoride and 3-n-propyl fluoride.

As used herein, the term "C1-6 alkoxy" refers to a group comprising the aforementioned "C1-6 alkyl" group and an oxy group. As examples there may be mentioned methoxy, ethoxy, isopropoxy and tert-butoxy groups.

As used herein, the term "C1-C6 alkylthio" refers to a group comprising the aforementioned "C6 alkyl" group and a thio group. As examples there may be mentioned methylthio and ethylthio.

As used herein, the term "C1-6 acyloxy" refers to a group comprising the aforementioned "C1-6 acyl" group and an oxy group. The term "C1-6 acyl" refers to a combination of the aforementioned "C1-6 alkyl" group and a carbonyl group, examples of which include acetyl, propionyl, butyryl, isobutyryl, valeryl, isovaleryl and pivaloyl. Examples of "C1-6 acyloxy" groups include acetoxy, propionyloxy, butyryloxy, isobutyryloxy, valeryloxy, isovaleryloxy and pivaloyloxy.

As used herein, the term "C1-6 acylamino" refers to a group comprising the aforementioned "C1-6 acyl" group and an amino group. As examples there may be mentioned acetylamino, propionylamino, butyrylamino, isobutyrylamino, valerylamino, isovalerylamino and pivaloylamino.

As used herein, the term "C1-6 halogenated alkoxy" refers to a group comprising a halogen atom and the aforementioned "C1-6 alkoxy" group. As examples there may be mentioned fluoromethoxy, 2-chloroethoxy, 1-bromoisopropoxy and 2-iodo-tert-butoxy.

In formula (VIII), Step 1 in which (VI) is synthesized from (VII) is a thiocarbamoylation reaction. As examples of reaction solvents there may be mentioned aprotic polar solvents such as N,N-dimethylformamide, N,N-dimethylacetamide, tetrahydrofuran or the like, as well as ester-based solvents such as ethyl acetate or nonpolar solvents such as toluene or n-hexane. As examples of bases there may be mentioned sodium hydride, sodium hydroxide, butyllithium or the like. The reaction temperature is between -50°C and 100°C. Particularly preferred is the use of an aprotic polar solvent (more preferably N,N-dimethylacetamide) as the solvent and sodium hydride as the base, with reaction between 0°C and 30°C (more preferably near 15°C).

The reaction of synthesizing (V) from (VI) as Step 2 in formula (VIII) is a step of Newman-Kwart rearrangement. Examples of reaction solvents that may be used include diphenyl ether, decalin, N,N-dimethylaniline, toluene, N,N-dimethylformamide, chloroform or the like. The reaction may also be carried out in the absence of a solvent. The reaction temperature is between 80°C and 280°C, and if necessary additives such as aluminum chloride, zinc chloride or the like may be included. For a shorter reaction time, it is most preferred to use diphenyl ether as the solvent and carry out heating between 200°C and 280°C (more preferably around 280°C).

Step 2 in formula (VIII) above must be carried out at high temperature, and from the viewpoint of industrial production safety, (V) may be synthesized from (IX) in Step 1A.

This step is a carbamoylation step. As examples of reaction solvents there may be mentioned aprotic polar solvents such as N,N-dimethylformamide, N,N-dimethylacetamide, tetrahydrofuran, t-butyl methyl ether or the like, as well as ester-based solvents such as ethyl acetate or nonpolar solvents such as toluene or n-hexane. Examples of bases that may be used include inorganic bases such as sodium hydride, potassium carbonate, sodium hydroxide, butyllithium or the like, and organic bases such as triethylamine, pyridine or the like. The reaction temperature is between -50°C and 100°C. It is most preferred to use an aprotic polar solvent (more preferably N,N-dimethylformamide, t-butyl methyl ether or tetrahydrofuran) as the solvent and sodium hydride, triethylamine or potassium carbonate as the base, with reaction between 0°C and 80°C (more preferably from about 20°C to 60°C).

The reaction of synthesizing (IV) from (V) as Step 3 in formula (VIII) is a step of ring closure reaction. As examples of reaction solvents there may be mentioned aprotic solvents such as tetrahydrofuran, t-butyl methyl ether, N,N-dimethylformamide, N,N-dimethylacetamide or the like, and ester-based solvents such as ethyl acetate. Examples of bases to be used as cyclization reagents include lithium diisopropylamide, sodium hydride, butyllithium or the like. The reaction temperature is between -30°C and 30°C. It is most preferred to use an aprotic solvent (more preferably tetrahydrofuran or t-butyl methyl ether) as the solvent and lithium diisopropylamide as the base, with reaction at 10°C or lower.

The reaction of synthesizing (I) and/or (III) from (IV) in Step 4 of formula (VIII) is a step of formylation under Vilsmeier conditions, generally known as a reaction for introducing formyl groups into aromatic compounds or aromatic heterocyclic compounds. Since compound (IV) is neither an aromatic compound nor an aromatic heterocyclic compound, formylation normally does not occur under these conditions. A phosphorus oxychloride or phosphorus oxybromide reagent is usually added at 1-5 equivalents, with a reaction temperature of between 0°C and 150°C. Most preferably, phosphorus oxychloride is added at 2-3 equivalents and reaction is conducted at between 80°C and 120°C (more preferably around 100°C). The reaction solvent is preferably N,N-dimethylformamide. Also, the necessary equivalent of N,N-dimethylformamide may be used with chloroform or dichloromethane as additional solvents. Upon completion of the reaction, the product may be obtained by extraction using a non-aqueous solvent, and adjusting the pH to 6 with aqueous sodium hydroxide or the like will allow compounds (I) and (III) to be obtained as crystals, which are more manageable, while also allowing compounds (I) and (III) to be obtained as a mixture.

In this step, a compound of formula (III) is produced in addition to a compound represented by formula (I), and this compound of formula (III) can be converted to a compound represented by formula (I) or formula (II) in the subsequent step without any particular need to separate formula (III) at this stage.

The production process of the invention therefore allows a formyl group to be introduced at the 3-position of benzo[b]thiophenes. In the process of the invention, formula (IV) can be used as the starting substance for formylation under Vilsmeier conditions.

The reaction of synthesizing compound (II) from a mixture of (I) and (III) in Step 5 of formula (VIII) is a reduction step. A metal hydride complex is preferably used as the reducing agent, with aluminum hydride complexes being particularly preferred. As solvents there may be mentioned tetrahydrofuran and the like, although alcohols such as methanol and ethanol may be added for increased reaction efficiency. The reaction temperature is between -30°C and 100°C. From the standpoint of industrial production safety, it is most preferred to use sodium bis(2-methoxyethoxy)aluminum hydride as the reducing agent for reaction in a toluene solvent at around 60°C.

When an aluminum hydride complex is not used for reasons of industrial production safety, the dehalogenating catalytic reduction for synthesis of (I) from (III) may be continued for reduction to synthesize (II) from (I).

In this case, the catalyst used for catalytic reduction to synthesize (I) from (III) is preferably a palladium catalyst such as 5-20% palladium-carbon, 20% palladium(II) hydroxide on carbon or the like. Such palladium catalysts may be dried or hydrous products, although hydrous catalysts are preferred from the viewpoint of industrial production safety. The hydrogen source may be hydrogen gas, formic acid, ammonium formate or the like, with hydrogen gas being preferred. As added bases there may be mentioned triethylamine, diisopropylamine or diethylamine, although diisopropylamine is especially preferred because it allows the amount of palladium catalyst to be reduced. As reaction solvents there may be mentioned tetrahydrofuran and alcohols such as methanol or ethanol, with tetrahydrofuran being especially preferred. The reaction temperature is between 0°C and 100°C.

In the reduction step for synthesis of (II) from (I), the reducing agent used is preferably a metal hydride complex. Sodium borohydride is especially preferred. As reduction solvents there may be mentioned tetrahydrofuran and the like, although alcohols such as methanol and ethanol may be added for increased reaction efficiency. The reaction temperature is between -30°C and 100°C.

The number of steps for formula (VIII) of the invention are 4 or 5, and therefore the process is shorter. The portable yield is a high yield of 54-76%.

A benzothiophene derivative of formula (II) produced by the process described above can be used to synthesize medically useful benzimidazole derivatives (for example, formula (XX)) according to, for example, the method described in International Patent Publication WO 01/53291; 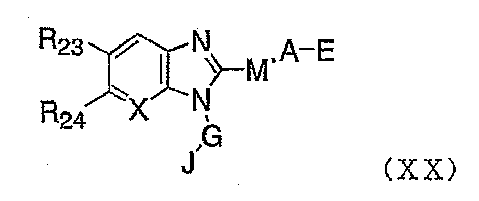 in formula (XX)
R₂₃ and R₂₄ simultaneously or each independently represent a hydrogen atom, a halogen atom, trihalomethyl, cyano, hydroxyl, C1-4 alkyl or C1-4 alkoxy, or R₂₃ and R₂₄ may together form -O-CH₂O-, -O-CH₂CH₂O- or -CH₂CH₂CH₂- (in which case the carbon atoms may be optionally substituted with one or more C1-4 alkyl groups);
A represents a substituted or unsubstituted C1-7 straight-chain, cyclic or branched alkylene group or alkenylene group, which may include one or more group(s) selected from the group consisting of -O-, -S-, -SO₂- and -NR₂₅- (where R₂₅ represents a hydrogen atom or a straight-chain or branched C1-6 alkyl group), and the substituent(s) on the groups may be a halogen atom or hydroxyl, nitro, cyano, straight-chain or branched C1-6 alkyl or straight-chain or branched C1-6 alkoxy groups (including cases where two adjacent groups form an acetal bond), straight-chain or branched C1-6 alkylthio, straight-chain or branched C1-6 alkylsulfonyl, straight-chain or branched C1-6 acyl, straight-chain or branched C1-6 acylamino, trihalomethyl, trihalomethoxy, phenyl, oxo or phenoxy optionally substituted with one or more halogen atom(s), and one or more of the substituents at any desired position of the alkylene or alkenylene group may be independently substituted, with the exclusion of cases where M in the formula is a single bond and hydroxyl and phenyl groups simultaneously substitute carbons of A bonded to M;
E represents -COOR₂₅, -SO₃R₂₅, -CONHR₂₅, -SO₂NHR₂₅, tetrazol-5-yl, 5-oxo-1,2,4-oxadiazol-3-yl or 5-oxo-1,2,4-thiadiazol-3-yl (where R₂₅ is as defined above);
M represents a single bond or -S(O)m-, where m is an integer of 0-2;
G and J together represent formula (II) above, with G representing the methylene group at position 3 of the benzothiophene of formula (II), and the hydroxyl group of formula (II) being substitute by the nitrogen atom on the benzimidazole ring; and
X represents -CH= or a nitrogen atom.

When E is COOR₂₅ and M is S in the benzimidazole derivative (XX), production may be carried out by Synthesis Scheme (A) or Synthesis Scheme (B) shown below. 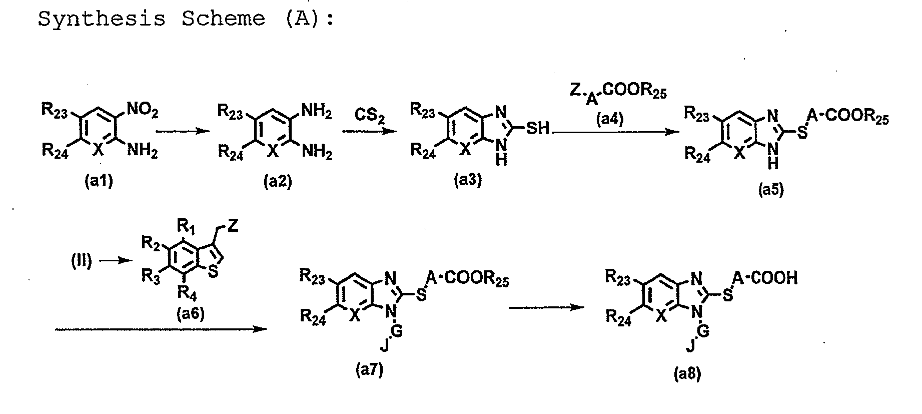 wherein Z represents a halogen, sulfonyloxy or an ammonium salt, and R₂₃, R₂₄, R₂₅, A, G, J and X are as defined above.

Specifically, the nitro group of a 2-nitroaniline derivative (a1) is reduced to obtain an orthophenylenediamine (a2). This is reacted with CS₂ to form compound (a3), after which a halide ester derivative (a4) is reacted therewith to obtain (a5) which is reacted with a halide derivative (a6) obtained by halogenating the hydroxyl group of formula (II), to obtain compound (a7). If necessary, this product may be further subjected to hydrolysis to obtain a benzimidazole derivative (a8) wherein R₂₅ is a hydrogen atom.

Reduction of the nitro group may be accomplished with ordinary catalytic reduction reaction conditions, such as reaction with hydrogen gas at a temperature of room temperature to 100°C under acidic, neutral or alkaline conditions in the presence of a catalyst such as Pd-C. A method of treatment using zinc or tin under acidic conditions or a method of using zinc powder under neutral or alkaline conditions may also be employed.

The reaction between the orthophenylenediamine derivative (a2) and CS₂ may be conducted by, for example, the methods described in J. Org. Chem. 1954 Vol. 19 p.631-637 (pyridine solution) or J. Med. Chem. 1993 Vol. 36 p.1175-1187 (ethanol solution).

The reaction between the thiobenzimidazole (a3) and halide ester (a4) may be conducted under ordinary S-alkylation conditions, and for example, by stirring at a temperature of 0°C-200°C in the presence of a base such as NaH, Et₃N, NaOH, K₂CO₃ or the like.

As halogenation reagents for conversion of the 3-hydroxymethyl-benzothiophene derivative (II) to (a6) there may be mentioned hydrogen halides, phosphorus halides, sulfonic acid chloride, thionyl halides and the like, among which phosphorus halides and thionyl halides are preferred, and phosphorus tribromide is especially preferred. As solvents there may be mentioned hydrocarbons such as cyclohexane and hexane and aromatic hydrocarbons such as benzene, toluene and xylene, among which cyclohexane and toluene are preferred. The reaction may be carried out from room temperature to reflux temperature, for several minutes to several hours.

The reaction between the thiobenzimidazole (a5) and halide derivative or ammonium salt (a6) may be conducted under ordinary N-alkylating or N-acylating conditions, and for example, by stirring at a temperature of 0°C-200°C in the presence of a base such as NaH, Et₃N, NaOH, K₂CO₃, Cs₂CO₃ or the like.

As the dissociation reaction for the carboxyl protecting group R₂₅, there is preferably employed a method of hydrolysis using an alkali such as lithium hydroxide or an acid such as hydrochloric acid or trifluoroacetic acid.

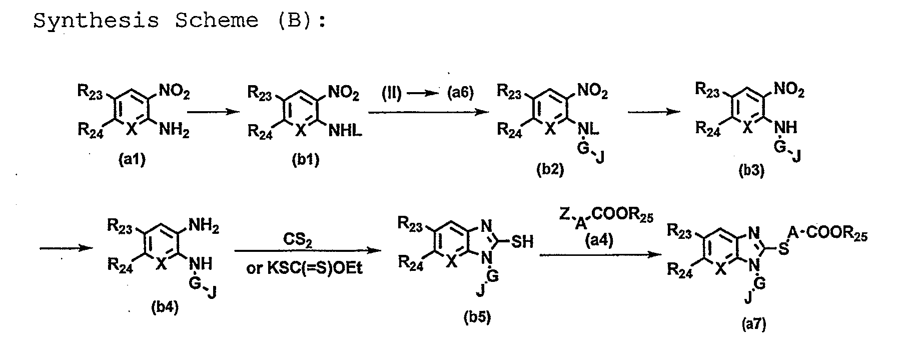

Specifically, the amino group of a 2-nitroaniline derivative (a1) is protected with an appropriate protecting group L to obtain (b1). This is reacted with a halide derivative (a6) obtained by halogenating the hydroxyl of formula (II) to obtain (b2), and L is deprotected to obtain (b3). The nitro group of (b3) is reduced to obtain an orthophenylenediamine derivative (b4). This is then reacted with CS₂ or KSC(=S)OEt to form compound (b5), which may be reacted with a halide ester derivative (a4) to obtain benzimidazole derivative (a7). If necessary, this product may be further subjected to hydrolysis to obtain a benzimidazole derivative wherein R₂₅ is a hydrogen atom.

Alternatively, the halide derivative (a6) may be reacted without protection of the 2-nitroaniline derivative (a1) to directly obtain compound (b3). The protecting group L may be trifluoroacetyl, acetyl, t-butoxycarbonyl, benzyl or the like. The reaction between the orthophenylenediamine derivative (b4) and CS₂ may be conducted in the same manner as in Synthesis Scheme (A), and the reaction with KSC(=S)OEt may be carried out by the method described in Organic Synthesis (OS) 1963, Vol. 4, p.569-570. The other reactions may be carried out in the same manner as in Synthesis Scheme (A).

When E is tetrazol-5-yl and M is S in the benzimidazole derivative (XX), production may be carried out by Synthesis Scheme (E) shown below. 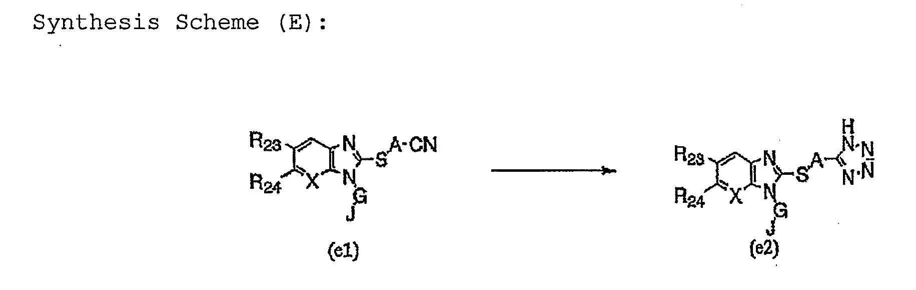 wherein R₂₃, R₂₄, A, G, J and X are as defined above.

A nitrile compound (e1) is reacted with an azide for conversion to a tetrazole compound (e2). As azides there may be mentioned trialkyltin azide compounds such as trimethyltin azide, and hydroazic acid or its ammonium salt. When an organic tin azide compound is used, it is preferably used in a 1-4 molar amount with respect to compound (e1). When hydroazic acid or its ammonium salt is used, sodium azide and ammonium chloride or a tertiary amine such as triethylamine are preferably used in amount a 1-5 molar amount with respect to compound (e1). Each reaction is carried out using a solvent such as toluene, benzene or DMF at a temperature of 0°C-200°C.

When M is SO or SO₂ in the benzimidazole derivative (XX), production may be carried out by Synthesis Scheme (F) shown below. 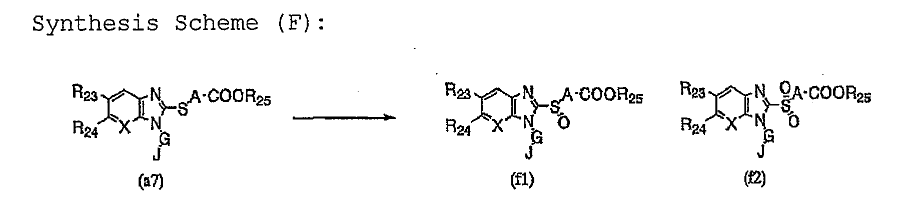 wherein R₂₃, R₂₄, R₂₅, A, G, J and X are as defined above.

Specifically, a benzimidazole compound (a7) is reacted with a peroxide compound in an appropriate solvent to obtain a sulfoxide derivative (f1) and/or sulfone derivative (f2). The peroxide compound used may be, for example, perbenzoic acid, m-chloroperbenzoic acid, peracetic acid, hydrogen peroxide or the like, and the solvent used may be, for example, chloroform, dichloromethane or the like. The proportion in which compound (a7) and the peroxide compound are used is not particularly restricted and may be selected as appropriate within a wide range, but for most purposes it is preferably between about a 1.2- and 5-molar amount. Each reaction is carried out at normally about 0-50°C and preferably between 0°C and room temperature, and is usually completed by about 4-20 hours.

When M in the benzimidazole derivative (XX) is a single bond, production may be carried out by Synthesis Scheme (G) shown below. 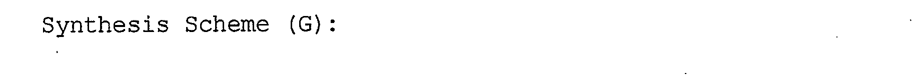 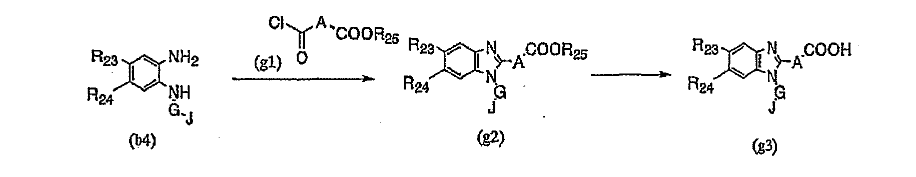 whrerein X, A, G, J and R₂₅ are as defined above.

Specifically, a known acid chloride derivative (g1) is reacted with a diamine compound (b4) to obtain a benzimidazole derivative (g2). If necessary, the -COOR₂₅ group of (g2) may be hydrolyzed to obtain a benzimidazole derivative (g3) wherein R₂₅ is a hydrogen atom.

The cyclizing reaction is described in J. Med. Chem. 1993 Vol. 36, p.1175-1187.

### Examples

The present invention will now be explained by the following examples, with the understanding that the invention is in no way limited by these examples.

### [Example 1]

### Synthesis of dimethyl-thiocarbamic acid O-(2,3-dimethylphenyl) ester (compound (XVI))

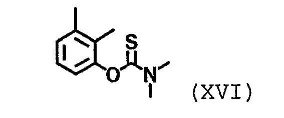

After charging 2,3-dimethylphenol (261 g, 2.14 mol), dimethylthiocarbamoyl chloride (291 g, 2.35 mol) and dimethylacetamide (1.3 L) in a reactor under an argon stream, the mixture was cooled to an internal temperature of below 15°C. Sodium hydride (94.0 g, 2.35 mol) was charged separately (over a period of 47 minutes) while stirring at an internal temperature of below 30°C, and upon completion of charging, the temperature was raised to room temperature. The mixture was stirred for 2 hours, and then sodium hydride (8.56 g, 0.214 mol) was further added prior to continued stirring for 2 hours. Upon confirming disappearance of the starting materials, quenching was performed with aqueous saturated ammonium chloride (1.3 L), and then ethyl acetate (1.0 L) and n-hexane (1.0 L) were added for re-extraction. The organic layers were combined, washed with water (1.3 L) and brine (1.3 L) and then dried over sodium sulfate, and the filtrate was concentrated under reduced pressure. The obtained crude product was purified by vacuum distillation (external temperature: 170°C, overhead temperature: 150°C, pressure reduction: 4.1 mmHg) to obtain compound (XVI) as white crystals (378.5 g, 1.81 mol) (yield: 84%).
¹H-NMR (300 MHz, CDCl₃) δ(ppm): 7.14-7.05(2H,m), 6.84(1H,d), 3.47(3H,s), 3.37(3H,s), 2.31(3H,s), 2.08(3H,s).

### [Example 2-1]

### Synthesis of dimethyl-thiocarbamic acid S-(2,3-dimethylphenyl) ester (compound XV)

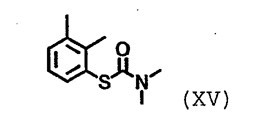

After charging dimethyl-thiocarbamic acid O-(2,3-dimethylphenyl) ester (378.5 g, 1.81 mol) as compound (XVI) and diphenyl ether (380 mL) in a reactor under an argon atmosphere, it was stirred at an external temperature of 275°C for 1 hour. Upon confirming disappearance of the starting materials, the mixture was cooled to room temperature, the diphenyl ether was first distilled off by vacuum distillation (external temperature: 130°C, overhead temperature: 105°C, pressure reduction: 3.3 mmHg), and then vacuum distillation was continued (external temperature: 160°C, overhead temperature: 140°C, pressure reduction 2.9 mmHg) to obtain compound (XV) as slightly yellow-tinted white crystals (343.0 g, 1.64 mol) (yield: 91%).
¹H-NMR (300 MHz, CDCl₃) δ(ppm): 7.34(1H,d), 7.19(1H,d), 7.08(1H,t), 3.05(6H,brd), 2.36(3H,s), 2.31(3H,s).

### [Example 2-2]

### Synthesis of dimethyl-thiocarbamic acid S-(2,3-dimethylphenyl) ester (compound XV)

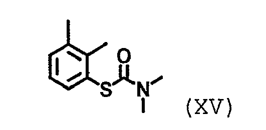

After charging sodium hydride (60% oil) (2.42 g, 60.6 mmol) and t-butyl methyl ether (11 mL) in a reactor under an argon atmosphere, a solution of dimethylbenzenethiol (5.58 g, 40.4 mmol) in t-butyl methyl ether (22 mL) and a solution of dimethylcarbamoyl chloride (4.78 g, 44.4 mmol) in t-butyl methyl ether (22 mL) were added dropwise in that order with an external temperature of 0°C, and the mixture was stirred at an external temperature of 60°C for 1.5 hours. Upon confirming disappearance of the starting materials, the mixture was cooled to room temperature, further cooled on ice and then neutralized and separated with 1M hydrochloric acid (28 mL). The aqueous layer was extracted with t-butyl methyl ether (28 mL), and the organic layers were combined and washed with aqueous 1M sodium hydroxide (56 mL), water (56 mL) and brine (56 mL). After drying the organic layer over sodium sulfate, the filtrate was concentrated under reduced pressure to obtain compound (XV) as a white solid (8.98 g, 40.9 mmol) (yield: 106%, including oil from sodium hydride). The obtained solid was used in the following step without further purification.

### [Example 2-3]

### Synthesis of dimethyl-thiocarbamic acid S-(2,3-dimethylphenyl) ester (compound XV)

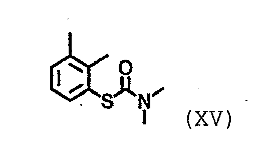

After charging dimethylbenzenethiol (0.89 g, 6.44 mmol), t-butyl methyl ether (4.5 mL) and potassium carbonate (1.78 g, 12.98 mmol) in a reactor under an argon atmosphere, a solution of dimethylcarbamoyl chloride (0.76 g, 7.08 mmol) in t-butyl methyl ether (4.5 mL) was added dropwise (washing twice with 4.5 mL of t-butyl methyl ether) at an external temperature of 0°C. The mixture was stirred at an external temperature of 60°C for 29 hours, and upon confirming disappearance of the starting materials, the mixture was cooled to room temperature, further cooled on ice and then neutralized and separated with 1M hydrochloric acid (10 mL). The aqueous layer was extracted twice with t-butyl methyl ether (4.5 mL), and the organic layers were combined and washed with aqueous 1M sodium hydroxide (9 mL), water (9 mL) and brine (9 mL). After drying the organic layer over sodium sulfate, the filtrate was concentrated under reduced pressure to obtain compound (XV) as a white solid (1.34 g, 6.42 mmol) (yield: 100%). The obtained solid was used in the following step without further purification.
¹H-NMR (300 MHz, CDCl₃) δ(ppm): 7.34(1H,d) 7.19(1H,d), 7.08(1H,t), 3.05(6H,brd), 2.36(3H,s), 2.31(3H,s).

### [Example 3-1]

### Synthesis of 4-methyl-3H-benzo[b]thiophene-2-one (compound XIV)

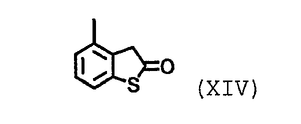

After charging tetrahydrofuran (1.29 L) and diisopropylamine (217 mL, 1.55 mol) in a reactor under an argon stream, the mixture was cooled to an internal temperature of -5°C. Upon confirming an internal temperature of near 0°C, n-butyllithium (1.0 L, 1.54 mol) was added dropwise over a period of 80 minutes at an internal temperature of below 5°C. Upon completion of the dropwise addition, stirring was continued at the same temperature for 1 hour and the mixture was cooled to an external temperature of -15°C. A solution of a dimethyl-thiocarbamic acid S-(2,3-dimethylphenyl) ester (128.9 g, 0.616 mol) as compound (XV) in tetrahydrofuran (387 mL) was then added dropwise (washing with 64 mL of tetrahydrofuran) over a period of 45 minutes at an internal temperature of below 0°C, and after stirring for 30 minutes, disappearance of the starting materials was confirmed. Next, 10% hydrochloric acid (1.93 L) was slowly added to the reaction mixture, the temperature was raised to near room temperature and the mixture was stirred for 30 minutes at near room temperature. Ethyl acetate (645 mL) was then added for extraction, and washing was performed with water (1.3 L x 3) and brine (1.3 L). After drying the organic layer over magnesium sulfate, the filtrate was concentrated under reduced pressure to obtain a crude light-yellowish white product (103.4 g) (crude yield: 102%).

Ethanol (47 mL) and n-hexane (188 mL) were added to 94.1 g of the crude product, and the mixture was heated to reflux at an external temperature of 100°C while confirming dissolution of the crystals. After stirring for 5 minutes the mixture was allowed to cool, and the crystals that precipitated at an internal temperature of near 40°C were stirred and then cooled on ice. After stirring for 1 hour at an internal temperature of 5°C, the crystals were filtered out and top-rinsed using a cooled ethanol/n-hexane mixture (ethanol/n-hexane = 1/4, 141 mL). The wet crystals were dried under reduced pressure at 50°C for 16 hours to obtain compound (XIV) as a slightly yellowish-white powder (72.55 g, 0.442 mol) (calculated yield: 79%).
¹H-NMR (300 MHz, CDCl₃) δ(ppm): 7.26-7.16 (2H,m), 7.03(1H,d), 3.83(2H,s), 2.27(3H,s).

### [Example 3-2]

### Synthesis of 4-methyl-3H-benzo[b]thiophene-2-one (compound XIV)

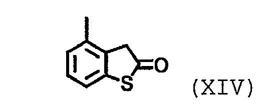

After charging t-butyl methyl ether (129 mL) and diisopropylamine (16.7 mL, 119 mmol) in a reactor under an argon atmosphere, the mixture was cooled to an external temperature of 0°C. Next, n-butyllithium (2.6M hexane solution, 45.9 mL, 119 mmol) was added dropwise over a period of one hour at an internal temperature of below 6°C. Upon completion of the dropwise addition, the mixture was stirred at the same temperature for 30 minutes and a solution of a dimethyl-thiocarbamic acid S-(2,3-dimethylphenyl) ester (10.0 g, 47.8 mmol) as compound (XV) in t-butyl methyl ether (60 mL) was added dropwise over a period of 2 hours at an internal temperature of below 4°C, and after further stirring for 30 minutes, disappearance of the starting materials was confirmed. Next, 3M hydrochloric acid (150 mL) was slowly added to the reaction mixture, the temperature was raised to near room temperature and the mixture was subjected to separation and extraction. The aqueous layer was extracted with t-butyl methyl ether (50 mL, twice), and the organic layers were combined and washed with water (100 mL, 3 times) and brine (100 mL). After drying the organic layer over magnesium sulfate, the filtrate was concentrated under reduced pressure to obtain a crude light-yellowish white product (7.8 g) (crude yield: 100%).

Ethanol (0.1 mL) was added to 1.0 g of the crude product for dissolution at an external temperature of 100°C, and then n-heptane (2.4 mL) was added at the same temperature. The mixture was subsequently cooled by standing and then by cooling on ice. After stirring for 1 hour at an internal temperature of 5°C, the crystals were filtered out and top-rinsed twice using a cooled ethanol/n-heptane mixture (ethanol/n-heptane = 1/24, 1.0 mL). The wet crystals were dried under reduced pressure at 50°C for 16 hours to obtain compound (XIV) as a slightly yellowish-white powder (0.84 g, 5.11 mmol) (calculated yield: 83%).
¹H-NMR (300 MHz, CDCl₃) δ(ppm): 7.26-7.16(2H,m), 7.03(1H,d), 3.83(2H,s), 2.27(3H,s).

### [Example 4-1]

### Synthesis of 4-methyl-benzo[b]thiophene-3-carbaldehyde (compound (XI)) and 2-chloro-4-methyl-benzo[b]thiophene-3-carbaldehyde (compound XIII)

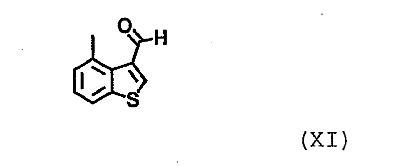

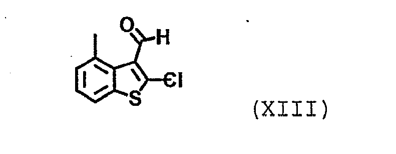

After charging 4-methyl-3H-benzo[b]thiophene-2-one (3.0 g, 18.27 mmol) as compound (XIV) and N,N-dimethylformamide (9 mL) in a reactor under an argon atmosphere, phosphorus oxychloride (3.72 mL, 40.19 mmol) was added dropwise over a period of 20 minutes at an external temperature of 0°C. After the dropwise addition, the external temperature was raised to 100°C, and after stirring for 2 hours, disappearance of the starting materials was confirmed. The mixture was then cooled to an external temperature of 0°C and aqueous 4.6M sodium hydroxide (35 mL) was slowly added for pH adjustment to 6, after which the mixture was stirred at room temperature for 10 minutes. The precipitated crystals were filtered out and top-rinsed with water (15 mL) to obtain a mixture of 4-methyl-benzo[b]thiophene-3-carbaldehyde (XI) and 2-chloro-4-methyl-benzo[b]thiophene-3-carbaldehyde (XIII) as a reddish-brown powder (wet, 3.22 g). The obtained wet crystals were used in the following step without purification or drying.
4-methyl-benzo[b]thiophene-3-carbaldehyde: ¹H-NMR (400 MHz, CDCl₃) δ(ppm): 10.42(1H,s), 8.43(1H,s), 7.75(1H,d), 7.33(1H,t), 7.28(1H,d), 2.84(3H,s).
2-chloro-4-methyl-benzo[b]thiophene-3-carbaldehyde: ¹H-NMR (300 MHz, CDCl₃) δ(ppm): 10.46(1H,s), 7.56(1H,d), 7.31(1H,t), 7.24(1H,d), 2.70(3H,s).

### [Example 4-2]

### Synthesis of 4-methyl-benzo[b]thiophene-3-carbaldehyde (compound (XI)) and 2-chloro-4-methyl-benzo[b]thiophene-3-carbaldehyde (compound XIII)

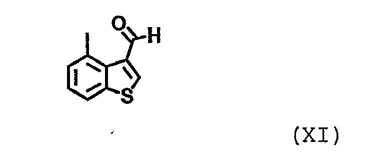

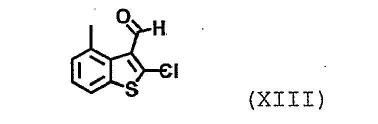

After charging 4-methyl-3H-benzo[b]thiophene-2-one (4.9 g, 29.7 mmol) as compound (XIV) and N,N-dimethylformamide (15 mL) in a reactor under an argon atmosphere, phosphorus oxychloride (10.0 g, 65.24 mmol) was added dropwise over a period of 30 minutes at an external temperature of 0°C. After the dropwise addition, the external temperature was raised to 100°C, and after stirring for 1.5 hours, disappearance of the starting materials was confirmed. The reaction mixture was then allowed to cool, toluene (24 mL) was added at 41°C and the mixture was then ice-cooled. Aqueous 5M sodium hydroxide (42 mL) was then slowly added dropwise and the mixture was subjected to separation and extraction. The aqueous layer was extracted three times with toluene (24 mL), and the organic layers were combined and washed with water (49 mL, 3 times) and brine (49 mL). After drying the organic layer over sodium sulfate, the filtrate was concentrated under reduced pressure to obtain a mixture of 4-methyl-benzo[b]thiophene-3-carbaldehyde (XI) and 2-chloro-4-methyl-benzo[b]thiophene-3-carbaldehyde (XIII) as a reddish-brown solid (5.42 g). The obtained wet crystals were used in the following step without purification or drying.

### [Example 5-1]

### Synthesis (4-methyl-benzo[b]thiophene-3-yl)-methanol (compound (XII))

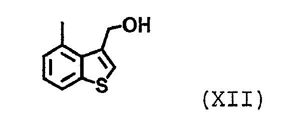

A mixture of compounds (XI) and (XIII) obtained in the previous step [Example 4-1] was used in half the charging amount. The weight and volume calculations were based on the wet weight of 4-methyl-benzo[b]thiophene-3-carbaldehyde and 2-chloro-4-methyl-benzo[b]thiophene-3-carbaldehyde, and the molar calculations were based on 4-methyl-3H-benzo[b]thiophene-2-one, i.e. a theoretical yield of 100% from the previous step.

A mixture of 4-methyl-benzo[b]thiophene-3-carbaldehyde and 2-chloro-4-methyl-benzo[b]thiophene-3-carbaldehyde (1.61 g wet weight, half from the previous step), tetrahydrofuran (8 mL), 5% palladium-carbon (160 mg, 50% wet) and diisopropylamine (3.11 mL, 22.2 mmol) were charged into the reactor, and the reaction system was substituted three times with argon and then three times with hydrogen. The mixture was stirred at an external temperature of 55°C for 9 hours under hydrogen at ordinary pressure, and upon confirming completion of the reaction, the mixture was allowed to stand at room temperature for 15 hours.

After standing, the reaction mixture was cooled to an external temperature of 0°C, and sodium borohydride (520 mg, 13.75 mmol) was added in small portions at a time. The mixture obtained from the addition was stirred at room temperature for 2 hours, and disappearance of 4-methyl-benzo[b]thiophene-3-carbaldehyde was confirmed. The reaction mixture was then cooled on ice, 1M hydrochloric acid (25 mL) was slowly added at an external temperature of 0°C, and extraction was performed with ethyl acetate (16 mL x 3). The organic layers were combined and washed with brine (16 mL) and dried over sodium sulfate, and then the filtrate was concentrated under reduced pressure to obtain (4-methyl-benzo[b]thiophene-3-yl)-methanol (compound (XII)) as a light orange-white powder (1.46 g, 8.19 mmol). No further purification was performed (portable yield: 90%).
¹H-NMR (400 MHz, CDCl₃) δ(ppm): 7.70(1H,d), 7.42(1H,s), 7.24(1H,t), 7.14(1H,d), 5.03(2H,s), 2.81(3H,s).

### [Example 5-2]

### Synthesis of (4-methyl-benzo[b]thiophene-3-yl)-methanol (compound (XIII))

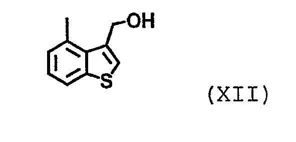

The mixture of 4-methyl-benzo[b]thiophene-3-carbaldehyde and 2-chloro-4-methyl-benzo[b]thiophene-3-carbaldehyde obtained in the previous step [Example 4-2] (1.00 g, 5.28 mmol based on theoretical 100% yield in previous step, i.e. 4-methyl-3H-benzo[b]thiophene-2-one) and toluene (5 mL) were charged into a reactor under an argon atmosphere, and sodium bis(2-methoxyethoxy)aluminum hydride (65% toluene solution, 2.46 g, 7.91 mmol) was added dropwise at an external temperature of 0°C. Upon completion of the dropwise addition, the mixture was stirred at an external temperature of 60°C for 24 hours, and upon confirming disappearance of the starting materials, the mixture was allowed to cool to room temperature, quenched with 1 mL of methanol and subjected to separation and extraction with aqueous 1M sodium hydroxide (5 mL). The aqueous layer was extracted three times with toluene (5 mL), and the organic layers were combined and washed with water (10 mL, 3 times) and brine (10 mL). After drying the organic layer over sodium sulfate, the filtrate was concentrated under reduced pressure to obtain compound (XII) as a light orange-white powder (0.85 g, 4.78 mmol). No further purification was performed (yield: 91%).

### Industrial Applicability

The 3-hydroxymethylbenzo[b]thiophene derivatives obtained by the production process of the invention can be used as production intermediates for drugs such as chymase inhibitors.

## Claims

1. A process wherein a compound represented by the following formula (I): 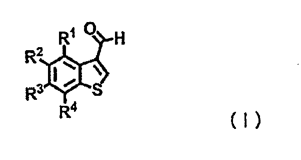 wherein R¹, R², R³ and R⁴ simultaneously or each independently represent a hydrogen atom, C1-6 alkyl, C1-6 halogenated alkyl, a halogen atom, cyano, C1-6 alkoxy, C1-6 alkylthio, C1-6 acyloxy, C1-6 acylamino or C1-6 halogenated alkoxy;
is reduced to produce a 3-hydroxymethylbenzo[b]thiophene derivative represented by the following formula (II) : 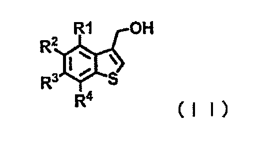 wherein R¹, R², R³ and R⁴ are as defined in formula (I).

2. The production process according to claim 1, wherein a metal hydride complex is used as the reducing agent.

3. The production process according to claim 2, wherein the metal hydride complex is sodium borohydride complex or aluminum hydride complex.

4. A process wherein a compound represented by the following formula (III): 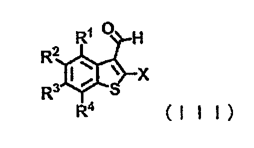 wherein R¹, R², R³ and R⁴ are as defined in formula (I), and X represents a halogen atom;
is reduced to produce a 3-hydroxymethylbenzo[b]thiophene derivative represented by formula (II).

5. The production process according to claim 4, wherein a metal hydride complex is used as the reducing agent.

6. The production process according to claim 5, wherein the metal hydride complex is an aluminum hydride complex.

7. The production process according to any one of claims 1 to 3, wherein the compound represented by formula (III) is dehalogenated to produce a 3-formylbenzo[b]thiophene derivative represented by formula (I).

8. The production process according to claim 7, wherein catalytic reduction is used as the dehalogenation conditions.

9. The production process according to claim 8, wherein hydrogen and palladium-carbon are used as the dehalogenation reagents.

10. A process wherein a mixture of a compound represented by formula (I) and a compound represented by formula (III) is reduced to produce a 3-hydroxymethylbenzo[b]thiophene derivative represented by formula (II).

11. The production process according to claim 10, wherein a metal hydride complex is used as the reducing agent.

12. The production process according to claim 11, wherein the metal hydride complex is an aluminum hydride complex.

13. The production process according to claim 12, wherein the aluminum hydride complex is sodium bis(2-methoxyethoxy)aluminum hydride.

14. The production process according to any one of claims 1 to 3 and 10 to 13, wherein a compound represented by the following formula (IV): 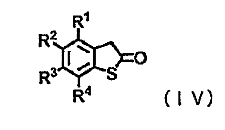 wherein R¹, R², R³ and R⁴ are as defined in formula (I);
is formylated to produce a 3-formylbenzo[b]thiophene derivative represented by formula (I).

15. The production process according to claim 14, wherein Vilsmeier reaction conditions are used as the formylation conditions.

16. The production process according to claim 14, wherein N,N-dimethylformamide and phosphorus oxychloride are used as the formylation reagents.

17. The production process according to any one of claims 4 to 13, wherein a compound represented by formula (IV) is formylated to produce a 3-formylbenzo[b]thiophene derivative represented by formula (III).

18. The production process according to claim 17, wherein Vilsmeier reaction conditions are used as the formylation conditions.

19. The production process according to claim 17, wherein N,N-dimethylformamide and phosphorus oxychloride are used as the formylation reagents.

20. The production process according to any one of claims 14 to 19, wherein a compound represented by the following formula (V): 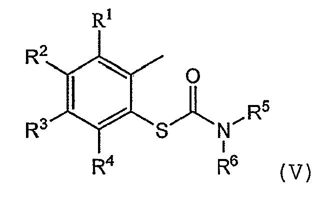 wherein R¹, R², R³ and R⁴ are as defined in formula (I), and R⁵ and R⁶ simultaneously or each independently represent a hydrogen atom, C1-6 alkyl, C1-6 halogenated alkyl, a halogen atom, cyano, C1-6 alkoxy, C1-6 alkylthio, C1-6 acyloxy, C1-6 acylamino or C1-6 halogenated alkoxy;
is cyclized to produce a dihydrobenzothiophene derivative represented by formula (IV).

21. The production process according to claim 20, wherein a base is used as the cyclizing reagent.

22. The production process according to claim 21, wherein lithium diisopropylamide is used as the cyclization reagent.

23. The production process according to any one of claims 20 to 22, wherein a compound represented by formula (IX): 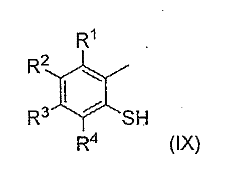 wherein R¹, R², R³ and R⁴ are as defined as in formula (I);
is reacted with a carbamoyl halide to produce a carbamoyl derivative represented by formula (V).

24. The production process according to claim 23, wherein a base is used as the reagent.

25. The production process according to claim 24, wherein potassium carbonate or sodium hydride is used as the reagent.

26. The production process according to any one of claims 20 to 22, wherein a compound represented by formula (VI): 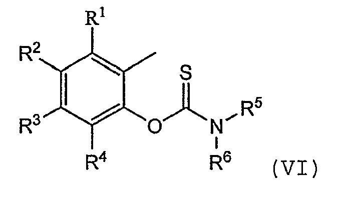 wherein R¹, R², R³, R⁴, R⁵ and R⁶ are as defined in formula (V);
is subjected to rearrangement reaction to produce a thiocarbamoyl derivative represented by formula (V).]

27. The production process according to claim 26, wherein the rearrangement reaction conditions include heating at a temperature of between 200°C and 300°C.

28. The production process according to claim 26 or 27, wherein a compound represented by formula (VII): 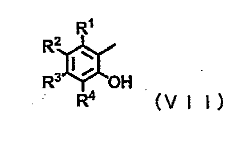 wherein R¹, R², R³ and R⁴ are as defined in formula (I);
is reacted with a thiocarbamoyl halide to produce a carbamoyl derivative represented by formula (VI).

29. A production process for benzimidazole derivatives represented by formula (XX), which includes the following production steps:
(i) a production process for a compound represented by formula (II), according to any one of claims 1 to 28; and
(ii) a process in which a benzimidazole derivative represented by formula (XX) is produced from the compound represented by formula (II); 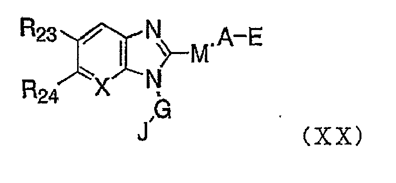 in formula (XX)
R₂₃ and R₂₄ simultaneously or each independently represent a hydrogen atom, a halogen atom, trihalomethyl, cyano, hydroxyl, C1-4 alkyl or C1-4 alkoxy, or R₂₃ and R₂₄ may together form -O-CH₂O-, -O-CH₂CH₂O- or -CH₂CH₂CH₂- (in which case the carbon atoms may be optionally substituted with one or more C1-4 alkyl groups);
A represents a substituted or unsubstituted C1-7 straight-chain, cyclic or branched alkylene group or alkenylene group, which may include one or more group(s) selected from the group consisting of -O-, -S-, -SO₂- and -NR₂₅- (where R₂₅ represents a hydrogen atom or a straight-chain or branched C1-6 alkyl group), and the substituent(s) on the groups may be a halogen atom or hydroxyl, nitro, cyano, straight-chain or branched C1-6 alkyl or straight-chain or branched C1-6 alkoxy groups (including cases where two adjacent groups form an acetal bond), straight-chain or branched C1-6 alkylthio, straight-chain or branched C1-6 alkylsulfonyl, straight-chain or branched C1-6 acyl, straight-chain or branched C1-6 acylamino, trihalomethyl, trihalomethoxy, phenyl, oxo or phenoxy optionally substituted with one or more halogen atom(s), and one or more of the substituents at any desired position of the alkylene or alkenylene group may be independently substituted, with the exclusion of cases where M in formula (XX) is a single bond and hydroxyl and phenyl groups simultaneously substitute carbons of A bonded to M;
E represents -COOR₂₅, -SO₃R₂₅, -CONHR₂₅, -SO₂NHR₂₅, tetrazol-5-yl, 5-oxo-1,2,4-oxadiazol-3-yl or 5-oxo-1,2,4-thiadiazol-3-yl (where R₂₅ is as defined above);
M represents a single bond or -S(O)m-, where m is an integer of 0-2;
G and J together represent formula (II) above, with G representing the methylene group at position 3 of the benzothiophene of formula (II), and the hydroxyl group of formula (II) being substituted by the nitrogen atom on the benzimidazole ring; and
X represents -CH= or a nitrogen atom.

30. A compound represented by the following formula (I): 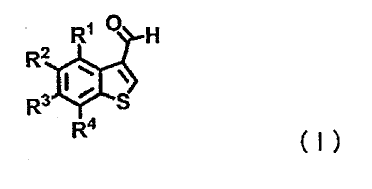 wherein R¹, R², R³ and R⁴ are as defined above.

31. The compound according to claim 30, wherein three among R¹, R², R³ and R⁴ are hydrogen atoms.

32. The compound according to claim 31, wherein R², R³ and R⁴ are hydrogen atoms.

33. The compound according to claim 32, wherein R¹ is a C1-6 alkyl group.

34. The compound according to claim 33 wherein R¹ is a methyl group.

35. A compound represented by the following formula (III): 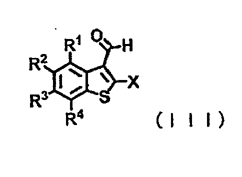 wherein R¹, R², R³ and R⁴ are as defined in formula (I).

36. The compound according to claim 35, wherein three from among R¹, R², R³ and R⁴ are hydrogen atoms.

37. The compound according to claim 36, wherein R², R³ and R⁴ are hydrogen atoms.

38. The compound according to claim 37, wherein R¹ is a C1-6 alkyl group.

39. The compound according to claim 38, wherein R¹ is a methyl group and X is a chlorine atom.

40. A compound represented by the following formula (V) : 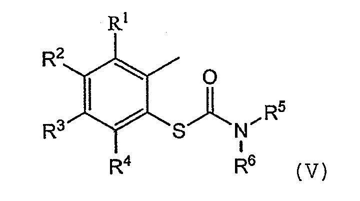 wherein R¹, R², R³, R⁴, R⁵ and R⁶ are as defined above.

41. The compound according to claim 40, wherein R⁵ and R⁶ simultaneously or each independently are methyl or ethyl groups.

42. The compound according to claim 41, wherein three from among R¹, R², R³ and R⁴ are hydrogen atoms.

43. The compound according to claim 42, wherein R², R³ and R⁴ are hydrogen atoms.

44. The compound according to claim 43, wherein R¹ is a C1-6 alkyl group.

45. The compound according to claim 44, wherein R¹ is a methyl group.

46. A compound represented by the following formula (VI) : 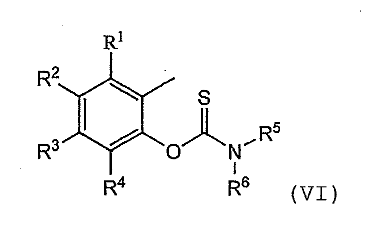 wherein R¹, R², R³, R⁴, R⁵ and R⁶ are as defined in formula (V).

47. The compound according to claim 46, wherein R⁵ and R⁶ simultaneously or each independently are methyl or ethyl groups.

48. The compound according to claim 47, wherein three from among R¹, R², R³ and R⁴ are hydrogen atoms.

49. The compound according to claim 48, wherein R², R³ and R⁴ are hydrogen atoms.

50. The compound according to claim 49, wherein R¹ is a C1-6 alkyl group.

51. The compound according to claim 50, wherein R¹ is a methyl group.
